(19) Europäisches Patentamt
European Patent Office
Office européen des brevets

(11) **EP 3 819 814 A1**

(12) **EUROPEAN PATENT APPLICATION**

(43) Date of publication:
**12.05.2021 Bulletin 2021/19**

(21) Application number: **19208146.1**

(22) Date of filing: **08.11.2019**

(51) Int Cl.:
*G06K 9/00* (2006.01)        *A61B 5/16* (2006.01)
*G06K 9/62* (2006.01)        *G06K 9/03* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(72) Inventors:
• **Asselborn, Thibault**
  **1005 Lausanne (CH)**
• **Johal, Wafa**
  **73200 Albertville (FR)**
• **Dillenbourg, Pierre**
  **1271 Givrin (CH)**
• **Lebourgeois, Corinne**
  **1131 Tolochenaz (CH)**

(71) Applicant: **Ecole Polytechnique Fédérale de Lausanne (EPFL)**
**1015 Lausanne (CH)**

(74) Representative: **Vesterinen, Jussi Tapio**
**LUMI IP GmbH**
**Rodtmattstrasse 45**
**3014 Bern (CH)**

(54) **METHOD OF ANALYSING HANDWRITTEN ITEMS**

(57)     The present invention according to one embodiment concerns a computer-implemented method of analysing handwritten characters produced by a user. The method comprises: collecting (107) handwritten characters of the user, the user being part of a given user group; extracting (109) a set of features from the handwritten characters, and allocating a numerical value for a respective feature in the set of features, a respective feature in the set of features characterising a respective handwriting aspect; determining (111) feature specific feature mean values and feature standard deviation values for the set of features of group members of the given user group; determining (113) feature score values for the set of features, a respective feature score value depending on a respective feature value, a respective feature mean value and a respective feature standard deviation value; and determining (115) a final handwriting score for the user from the feature score values.

Fig. 3

101 — Define handwriting features
103 — Train artificial intelligence system by using training data
105 — Extract feature importance values from artificial intelligence system
107 — Collect handwritten characters
109 — Extract features from handwritten characters and allocate numerical values for features
111 — Determine feature specific feature mean and standard deviation values based on pre-collected samples from users
113 — Determine feature score values based on numerical feature values, feature mean values and standard deviation values
115 — Determine final score based on feature score values
End

EP 3 819 814 A1

**Description**

TECHNICAL FIELD

The present invention relates to a method of analysing handwritten items, such as characters, text or drawings. More specifically, the analysis considers a set of handwriting features, where a given feature characterises a given aspect of the handwriting. Thus, the method may not only consider the appearance of the handwritten items, but also the manner how these items are produced. The invention also relates to a corresponding apparatus and system configured to carry out the method.

BACKGROUND OF THE INVENTION

Handwriting is a complex task involving cognitive, perceptual, attentional, linguistic, and fine motor skills. A major breakthrough in the understanding of handwriting has followed from the development, in the last decades, of graphic tablets and dedicated software programs, which enable high frequency sampling and subsequent analysis of handwriting dynamics. These tools greatly contributed to our current knowledge of handwriting kinematics and of the development of abilities for planning and controlling handwriting movements.

Until now, two main approaches have been used to analyse the quality of handwriting or to confirm the recognition of handwriting. The first one is a global holistic approach which evaluates the handwriting quality as a whole, while the second one measures it according to several predefined criteria. The global holistic approach is used to give an overall judgment of the quality of handwriting by comparing it with handwriting samples previously sorted according to their quality. Assessment of the writing quality only relies on a subjective judgement made by a human-being. The second commonly used approach for handwriting legibility analysis is based on predefined criteria (e.g. letter form, letter size, spacing and line-straightness). The judgment is then made by grading individually all these criteria and summing all these sub-scores. However, the observation of all these criteria still remains partly subjective because the assessment is carried out by a human-being.

Thanks to the emergence of new tools (e.g. tablet computers), the addition of several variables (hidden so far) to the analysis of handwriting legibility has become possible. In particular, the analysis of dynamic features of handwriting enables a better overall analysis. Several techniques taking into account the dynamics of handwriting have been proposed to classify the handwriting legibility, thus leading to better accuracy and reduced subjectivity.

Since the emergence of machine learning, a lot of research has been conducted to generate models for handwriting analysis. Indeed, machine recognition of handwriting has been used in various fields, such as reading postal addresses on envelopes, amounts in bank checks or signature verification. Models are generally divided into offline and online recognition. In the offline recognition, models focus on the appearance (image) of the handwritten text, while in the online recognition, the location of a tip of a pen is followed as a function of time.

Offline systems are generally less accurate than online systems due to the absence of temporal data containing information that may be relevant for the model. Only the image of the handwritten text is available to the model. On the contrary, for the online recognition, the temporal information about handwriting is available to the model. Different architectures of models may then be used for the classification. Currently, many models exist to recognise characters, words or sentences. However, the current solutions are not able to satisfactorily assess handwriting legibility or to confirm the correctness of the character recognition.

SUMMARY OF THE INVENTION

It is an object of the present invention to overcome at least some of the problems identified above related to analysing handwriting and/or to confirming the recognition of handwritten items.

According to a first aspect of the present invention, there is provided a method of analysing hand-drawn or handwritten items as recited in claim 1.

The proposed new solution has the advantage that the proposed method allows reliably analysing handwritten items, such as characters, or confirming the correctness of handwritten items. In other words, the quality of the handwritten items can be determined with high accuracy and an overall or final quality score can be obtained very quickly, i.e. typically within seconds or even quicker after the start of the analysis. The method obtains the final score from feature scores, where the feature scores may be determined for various features from different feature categories, including e.g. one or more of the following feature categories: a static feature category, a kinematic feature category, a pressure feature category and a tilt feature category. Thus, the method may not only consider traditional static features but also the dynamics of producing the items, the pressure applied to a writing surface by a writing instrument and/or tilt related aspects of the writing instrument may be taken into account in the analysis if so desired. The analysis can thus be carried out quickly and the results are completely objective as the analysis is carried out by a data processing apparatus.

According to a second aspect of the invention, there is provided a data processing apparatus for carrying out the proposed method as recited in claim 15.

Other aspects of the invention are recited in the dependent claims attached hereto.

BRIEF DESCRIPTION OF THE DRAWINGS

Other features and advantages of the present invention will become apparent from the following description of a non-limiting example embodiment, with reference to the appended drawings, in which:

• Figure 1 is a simplified view of the data collection setting according to an example of the present invention;

• Figure 2 is a simplified block diagram of a data processing device for carrying out the proposed method according to an example of the present invention;

• Figure 3 is a flow chart summarising the proposed character analysis method according to an example of the present invention; and

• Figure 4 is a plot showing example feature values for members of a given user group.

DETAILED DESCRIPTION OF AN EMBODIMENT OF THE INVENTION

An embodiment of the present invention will now be described in detail with reference to the attached figures. This embodiment is described in the context of analysing one or more handwritten characters, and more specifically alphabet letters and numbers (which may give an indication how well they are written), but the teachings of the invention are not limited to this environment. The teachings of the invention are equally applicable in any system configured to analyse or confirm the correct recognition of any characters or items, such as drawings, more broadly. In the present description, the word "character" is understood to cover any types of signs, glyphs or symbols, covering thus for example various alphabet letters and/or numbers but covering also a combination of individual symbols (words) even if they are separated by a word divider, such as a blank space (forming sentences). Handwritten items on the other hand are understood to cover any types of characters or drawings or shapes drawn by the user. Identical or corresponding functional and structural elements which appear in the different drawings are assigned the same reference numerals.

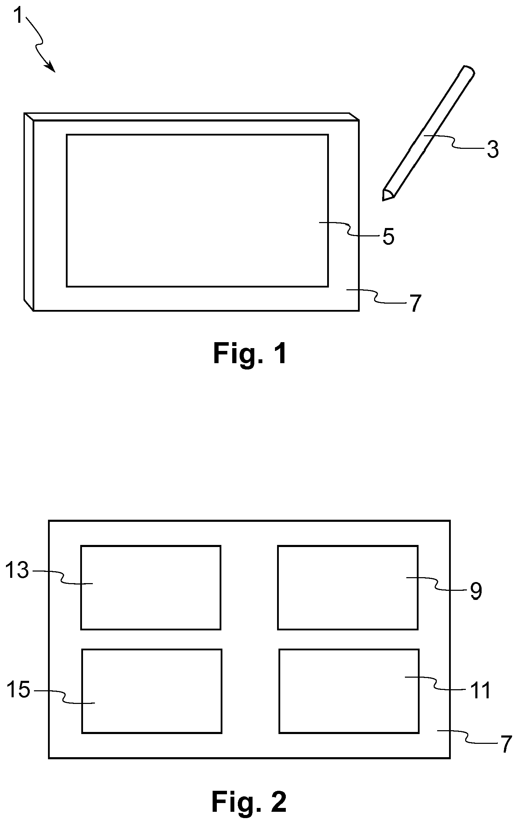

Figure 1 schematically illustrates a data collection system 1 or setting, which may be used to collect characters to be analysed or evaluated. In the present example, the items to be analysed are handwritten characters, such as letters and/or numbers or (numerical) digits (i.e. numeric symbols), referred to collectively also as glyphs. In this example, these characters are produced by a human-being with a writing instrument 3, such as a pen or pencil. Figure 1 also shows a piece of paper 5 placed on a sensitive surface, such as a touch screen surface (i.e. a tactile surface), of a data processing device or apparatus 7, such as a tablet computer. In this example, the data processing device is configured to carry out the proposed method. The characters may be written on the piece of paper or directly on the sensitive surface without using the paper.

Figure 2 is a simplified block diagram of the data processing device 7. The data processing device 7 comprises a data collection unit 9 for sensing or collecting the characters produced by a user. The collected characters may then be saved or stored in a memory unit 11, also referred to as a memory, before they are analysed by a hand-drawn or handwritten item analysis unit 13 (in this example operating as a character analysis unit), which in this example also comprises an artificial intelligence (AI) or machine learning unit or system as explained later in more detail. The analysis results from the item analysis unit 13 may also be saved in the memory unit 11. The data processing device 7 also comprises a central processing unit 15 for centrally controlling the operation of the data processing device 7.

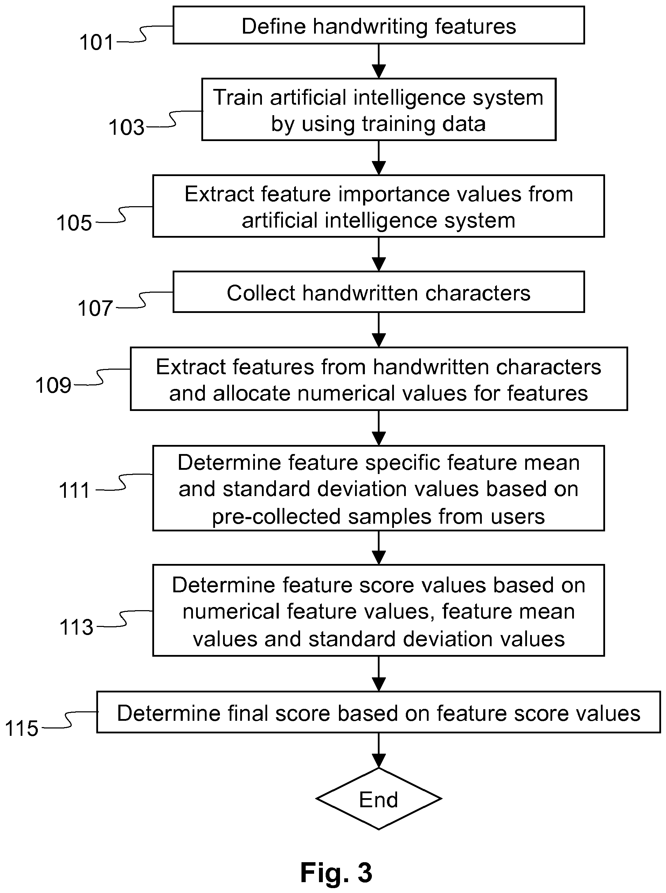

The hand-drawn or handwritten item analysis method is below explained in more detail with reference to the flow chart of Figure 3. It is to be noted that the analysis results may be used for example for training purposes to improve the user's handwriting. The results may also be used for example to reproduce digitally the characters input by the user once they have been properly analysed, i.e. interpreted optionally by imitating the handwriting style of the user. The result of the analysis may instead be used to verify the identity of the person entering the characters. In this case, the analysis result may be compared to stored characters or their average or mean, and the identity of the person writing the characters is only confirmed if the analysis result (e.g. an intermediate or final score) exceeds a pre-defined quality criterion, such as a threshold value. In this case, the analysis result may be used to verify a handwritten password (or any other hand-produced shape) entered by the user. The password may be approved only if both the entered word is correct but also the manner how it was written. This kind of double verification process increases security for instance when used in an access control process to grant access to a restricted domain, such as a computing device, a building, a restricted area, a user account, a service etc.

In step 101, a set of handwriting features are defined. Each handwriting feature characterises one or more handwriting aspects. The handwriting features may be classified into one or more feature classes. In this example, the handwriting features can be divided into four distinct feature classes, namely:

- static features: purely geometric characteristics of a written text;

- kinematic features: dynamics of handwriting path;

- pressure features: characteristics of the pressure recorded between the writing instrument tip and the writing surface; and

- tilt features: characteristics of the pen tilt.

The number of different features may be freely chosen such that the number may for instance be between 10 and 500, or more specifically between 50 and 150. In this way, and contrary to many existing techniques, the proposed method may take into account not only the final static product of handwriting but also any one of the following aspects: dynamic, pressure and tilt related aspects. It has been discovered that the final handwriting product is sometimes not enough to properly assess the quality of handwriting.
These four feature classes and their possible content is next explained in more detail.

Static features

The static features may be divided into two main categories. A first category regroups features which assess handwriting quality at a letter level. A direct translation of these features requires knowledge of the letter's shape. Since this would require a large-scale analysis of shapes of letters, which would be language-dependent due to variations in the Latin alphabet, these features are disregarded in the present example analysis. However, it would be also possible to consider these features in the analysis. A second category of features focuses on higher-level aspects of handwriting as briefly explained below.
Space between words: The distance (in pixels) between words, averaged for the entire text.
Handwriting density: A grid with a given number of pixel cells (such as 300 pixel cells) covering the entire range of the handwriting trace can be created. The number of points in each cell, if present, are stored in an array. The mean value of this array represents an approximation of the handwriting density.
Moment of handwriting: To compute this feature, bins of a given number of points, such as 300 points (from the same line of text), can be extracted, and their barycentre computed. The distance in the y direction (i.e. in the vertical direction) between consecutive barycentres is computed and averaged for all of the points. This reflects the average direction of the written line.
Handwriting size: To compute this feature, bins of a given number of points, such as 300 points (from the same line of text) can be extracted, and the total surface occupied by the box bounding the trace can then be computed.
Tremor frequencies: This feature quantifies shaky handwriting. For each user, the signal can first be divided into bins of a given number of points, such as 600 points, and from each of these bins the deviation from the handwriting path can be extracted. To do so, two types of vectors are extracted: for the first one, a "global" vector is computed by averaging bins of a given number of points, such as 10 points. This vector represents the global direction of the handwriting movement in a restricted area of 10 points. The second vector is local as it is not averaged over bins of points. It simply links points inside this restricted area of 10 points. The cross product of these two vectors indicates how orthogonal the local vector is compared to the "global" vector. The greater the result of this operation is, the higher the deviation from the path is. It can be determined that shaky handwriting will result in local vectors being rarely aligned with their global counterparts and can then be detected with this method. For each of the 600 points, the norm of the cross product can be saved.
Fourier transform can then be computed on the vectors, regrouping the results of all of these cross products. Then, the average of all of the Fourier transforms coming from these different bins of 600 points can be computed. In this manner, a normalisation is finally achieved for every user in the database. With this analysis, it was aimed to quantify the tremor/shaky aspect of handwriting, which would then be translated by higher frequencies or a wider bandwidth in the spectral domain. For example, the range of frequencies covering 90% of the spectral density can be extracted. It can thus be concluded that the smaller this value is (meaning that the distribution is more clustered), the more proficient the writer is. A writer having a huge bandwidth will not be fluent as they are less consistent in their movements. This feature is called "bandwidth of tremor frequencies". Motivated by this concept, the median of the power spectral density can also be extracted. A higher value of this feature indicates a higher presence of high frequencies. This feature is referred to as "median of power spectral density of tremor frequencies.

The last feature defined in this context is the distance between the spectral distribution of the writer to the averaged spectral distribution of all the writers in the database. The higher this distance is, the more eclectic the handwriting of this particular writer is. This feature is called "distance to mean of tremor frequencies".

Kinematic features

Handwriting speed: It can be hypothesised that abnormal variability in speed is indicative of handwriting problems. The speed can be quantified as the distance traveled by the writing instrument divided by the time taken. Although data are collected at 200 Hz, high frequency noise was detected, and, to remedy this issue, a moving average filter with $n$ =10 can be applied and then subsampled at every 10th point. The measurement is only kept if the writing instrument stayed on the surface during the 10 points (no in-air time). Finally, the mean, maximum, and standard deviation for each user are computed.

With this technique, the local handwriting speed can be accessed every 10 points. A linear regression can then be performed to compute the evolution of the handwriting speed. Motivated by insights from clinicians, the number of speed peaks per seconds can also be computed. To that end, a Gaussian filter is applied to the signal of velocity over time, and the number of local maxima and minima are computed. The number of peaks can be expected to grow with the total duration of the test, and, therefore, this number is normalised by time.

Handwriting speed frequencies: Handwriting can be interpreted as a two-dimensional time series (i.e. speed as a function of time). As such, common time-series analysis techniques can be applied, and in particular, the Fourier transform is computed. The process described in connection with the tremor frequency calculation is conducted and then the "bandwidth of speed frequencies", the "median of power spectral density of speed frequencies", and the "distance to mean of speed frequencies" are extracted.

Handwriting acceleration: Acceleration is another measure of variability in speed. The mean, maximum, and standard deviation of acceleration can be computed following the same procedure as that used to extract the mean, maximum, and standard deviation of handwriting speed.

In-air time ratio: The in-air time ratio represents the proportion of time spent by the writer without touching the surface of the writing surface. This was found to be a discriminative feature for the analysis of handwriting quality.

Pressure features

Pressure: The first features concerning the pressure are simply the mean, maximum, and standard deviation of the pressure.

Speed of pressure change: To compute the speed of pressure change, the same method as is used for the speed of handwriting can be used. For example, it is possible to work with averaged buckets of 10 points and divide the time spent by the difference between these two averaged bins of points. The mean, maximum, and standard deviation of these measures can then once again be extracted. The number of peaks of speed of pressure change during handwriting is also extracted. A Gaussian filter is applied to the signal and local minima and maxima of this filtered signal are extracted and normalised by the total amount of handwriting time (excluding the in-air time).

Speed of pressure change frequencies: The speed of pressure change can be seen as a time series, and frequencies can be extracted using a Fourier transform. The same process as that described in connection with the tremor frequency calculation is followed to extract the "bandwidth of speed of pressure change frequencies", the "median of power spectral density of speed of pressure change frequencies", and the "distance to mean of speed of pressure change frequencies".

Tilt features

The data measuring the writing instrument tilt with two different angles can be logged, which can be referred to as tilt-x and tilt-y angles. Both angles are measured for instance in the range between -60° and 60°. The tilt-x reflects the inclination of the writing instrument in the direction of the written line, and the tilt-y reflects the inclination of the writing instrument below the written line.

Tilt: Simple features can be extracted for both angles, namely the mean, maximum, and standard deviation of the measurement.

Speed of tilt change: The speed of tilt-x/tilt-y change can be computed in the same way as above, and the mean, maximum, standard deviation, and number of peaks are extracted. Finally, the evolution of the speed of tilt-x/tilt-y change is computed overtime.

Frequency of speed of tilt change: Using the same method as above, the "bandwidth of speed of tilt change frequencies", the "median of power spectral of speed of tilt change frequencies", and the "distance to mean of speed of tilt change frequencies" can be computed.

It is to be noted that merely some examples of possible handwriting features were briefly explained above. The number of different features used in the present method may be freely chosen. The number of different features may for instance be between 10 and 500, or more specifically between 50 and 150. Referring again to the flow chart of Figure 3, in step 103, the AI system is trained with handwriting features coming from data extracted from various users from a training data set. These users in this example are divided into different user groups according to the users' gender, age and/or laterality, i.e. left or right handedness. The present example uses a random forest model (RF) for the AI system although the present invention is not limited to RFs. For instance, an artificial neural network, such as a convolutional neural network or a recurrent neural network, could be used instead. Pre-computed features (with numerical values allocated to the features) extracted from handwriting samples at different time steps can be used as inputs for the RF model. As mentioned above, these features can capture various aspects of handwriting including static aspects, kinematics, pressure and tilt related aspects. In this way, and contrary to many existing techniques, the proposed method may take into account not only the final static product of handwriting but also the manner how the characters are produced.

An RF model consists of a large number of individual decision trees that operate as an ensemble. Each individual tree in the random forest outputs a class prediction (for example if handwriting problems are detected or not) and the class with the most votes becomes the model's prediction. The rules encoded within the decision tree can be learnt from the data via machine learning techniques. Practice has proven that RFs may be successfully employed to solve various practical problems.

The data set used to train the RF model or any other suitable AI system can be split into two distinct datasets: a first data subset, referred to as a training data set, and a second data subset, referred to as a test data set or validation set. In this example, the training data set represents 80% of the data samples of the data set, while the test data set represents 20% of the data samples of the data set. In order to give more statistical power to the learning procedure, a k-fold cross validation may be used for the learning procedure. In this way, it is possible to have an idea how reliable/stable the results are by comparing the results obtained by using k different training models. A k-fold (with k=5 for example) cross-validation was performed for the training. For each fold, the ratio between the training data set size and test data set size was fixed to 80% - 20%. Since k was set to 5 and 20% of the data were used for the test in each run, every sample giver in the database was used in the test set exactly once. The data samples of the data set comprise features, extracted from handwriting samples from a random set of people (i.e. samples from different people) of a given target group. Different target groups may be distinguished from each other by the age, gender and/or handedness of the sample givers in the groups.

The training data set is used to train the RF model during a training phase by pairing the input with expected output, while the test data set is used during a test phase to estimate how well the RF has been trained and/or to verify that the trained RF operates correctly. The training data comprise hand-drawn or handwritten items, and in this example, the training data consider the manner how the items are generated and also the appearance of the items. In this example, the data samples of the training data set comprise equal number of features for different handwriting samples. The larger the training data set it, the more reliably the proposed method can analyse any handwriting sample or to correctly detect handwriting problems from a sample written by any user.

Once the model is trained, in step 105, the features' importance for the analysis result is determined using any suitable technique. In this example, the importance is based on the Gini importance by e.g. following the teachings of "A comparison of random forest and its Gini importance with standard chemometric methods for the feature selection and classification of spectral data", Bjoern H Menze, et al., BMC Bioinformatics, volume 10, (2009). This method thus allows extracting the importance of any feature for the analysis or prediction. The feature importance values are in this example determined by using the training data and the test data separately. If for example, the aim of the prediction model is to determine whether or not a user presents handwriting problems, the RF model can find out what the most important features are for determining handwriting problems from a handwriting sample.

In step 107, the user enters the handwritten characters to be analysed. In other words, in step 107, the data processing device 7 collects the handwritten characters during a collection period. It is to be noted that each character may be understood as a trajectory, i.e. a sequence of consecutive positions of the tip of the writing instrument on the writing surface. The present example method takes into account the temporal aspect of data and thus considers the dynamics of writing. It is to be noted that certain characters may look very similar if only the final trace is inspected. This is for example the case between the letter "e" and the letter "l" or between the letter "g" and the number "9".

In step 109, the data processing device 7 extracts features from the handwritten characters. In other words, a set of features is extracted, where the set may be a subset of the features defined in step 101. Alternatively, the number of features extracted in this step may equal to the number features defined in step 101. When extracting the features, numerical values are allocated to the extracted features. It is to be noted that the value allocation and the feature extraction could instead be carried out as two separate steps, but in this example, the features are extracted by allocating the numerical values to the extracted features.

In step 111, feature mean values and feature standard deviation values are computed or determined for the features in the set of features based on all available user data or based on a subset of that data. However, the currently input characters or the user data of the present user are not used in this example to determine the feature mean values and feature standard deviation values. The standard deviation $\sigma$ is obtained for a given population according to the following equation:

$$\sigma = \sqrt{\frac{1}{N}\sum_{i=1}^{N}(x_i - \mu)^2},$$ (1)

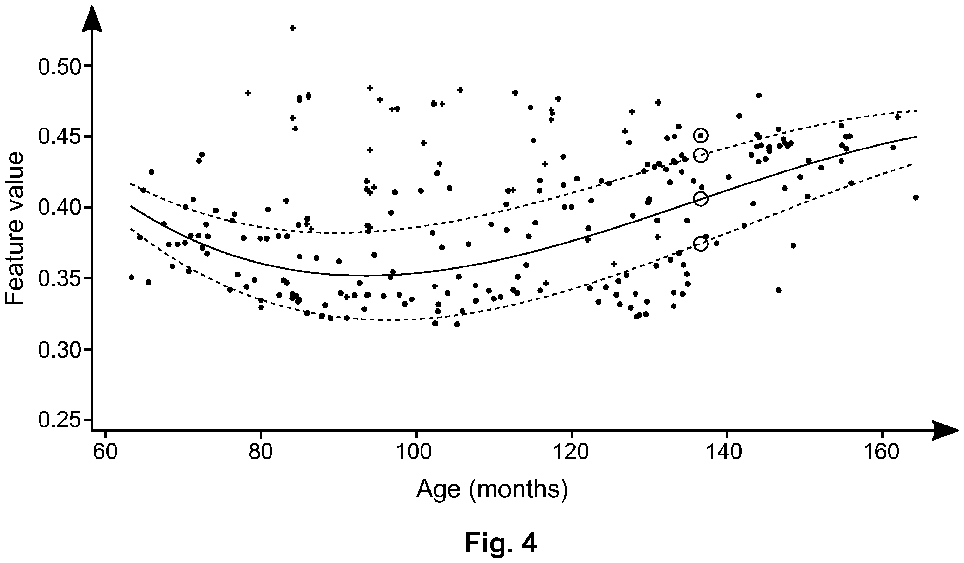

where the mean $\mu = \frac{1}{N}\sum_{i=1}^{N} x_i$, $N$ denotes the number of samples in the population, and $x_i$ are the observed samples in the population or their values, and $\frac{1}{N}\sum_{i=1}^{N}(x_i - \mu)^2$ is the variance. It is to be noted that instead of determining standard deviation values or any values directly derivable from them, it would be possible to determine variance values or any other parameter values related to the spread of a data distribution, where the data distribution consists of group members (or their feature values) that advantageously belong to the same user group as the user in question. Instead of carrying out step 111 at this moment, it could be carried out at any moment before step 113. The obtained mean and standard deviation values (or any suitable feature distribution related parameter values) are feature specific. It is to be noted that feature specific mean and standard deviation values are determined separately for a given gender, age group and/or left and right-handed users. In other words, for each feature and for both genders and handednesses separately, the mean of a given feature, as well as the standard deviation as a function of age can be computed as can be seen in the example of Figure 4. The solid line indicates the mean or average value as a function of time for a given user group, while the dashed lines indicate the standard deviation for the given user group (e.g. right-handed female users). The dots indicate users of a first subgroup while the crosses indicate users of a second subgroup within the given group of users. The circles indicate an example use case for a particular user for determining the mean and standard deviation values from the graph for that particular user. The feature specific mean value and standard deviation value can then be used to compute or obtain a deviation value for each of the features in the set of features as shown in Equation 2 below:

$$deviation = |(feature - f_{mean}(age))/f_{std}(age)|,$$ (2)

where *feature* indicates the value of the feature, $f_{mean}(age)$ is a function describing the feature's average or mean as a function of age, and $f_{std}(age)$ is a function describing the feature's standard deviation as a function of age. It is to be noted that $f_{std}(age)$ could be replaced by $f_{spread}(age)$ denoting a spread related function for the given data distribution (of the given user group).The deviation value indicates how far a given feature value is from corresponding feature values from other users in the given group of users. The deviation value can then be used in step 113 to obtain a feature score for a given feature. The score measures the proficiency of the user in a specific aspect of handwriting described by the extracted feature. In other words, the feature score indicates the user's proficiency for that feature. The feature score can be computed or obtained as shown in Equation 3 below:

$$score = e^{-0.15 \times deviation}.$$ (3)

The score obtained by Equation 3 leads to a score value between 0 and 1. The higher the score is for a given feature, the higher the quality of the given feature is determined to be. However, it is to be noted that there are alternative ways to determine the score. More broadly, the score is a function of the deviation (or the spread parameter), i.e. *score* = *f*(*deviation*) or *score* = $e^{(deviation)}$. In the above equations *e* refers to an exponential function, such that $f(y) = ab^y$, where *e* = *ab,* and *a* and *b* are positive real numbers, and in which the argument *y* occurs as an exponent.

It has been discovered that some features are more important than other features for describing the quality of handwriting or for determining handwriting problems. In that sense, it would be advantageous to extract the importance of each feature in the feature set in order to compute a global or final quality score. In the present example, the feature importance values were determined in step 105. In step 115, the feature importance values are used for obtaining a final score for the handwritten characters. More specifically, in this example, the sum of all the features multiplied by their respective importance is used for obtaining the final score as given in Equation 4 below:

$$finalscore = \sum_{i}^{n} score_{feature_i} \times importance_{feature_i},$$ (4)

where *i* denotes the feature index, *n* denotes the number of features to be considered, $score_{feature_i}$ denotes the score for feature *i,* and $importance_{feature_i}$ denotes the importance value of feature *i*. The final score as obtained in Equation 4 can then optionally be divided by *n.* then All the values used in Equation 4 are in this example positive real numbers. In this specific example, the final score ranges from 0 to 1, where 0 means the worst handwriting quality while 1 means the best handwriting quality. The above computation of the final score thus considers the feature's discriminative power. However, it is to be noted that it is not necessary to use any weights in the computation, as the final score may also be obtained by simply summing the feature scores of the features of the set of features without any weighing (and then optionally averaging the sum). Instead of using Equation 4 to obtain the final score, a machine learning or artificial intelligence algorithm, such as a k-means clustering based algorithm, may be used such that the feature score values are used as input values for the algorithm, which is then able to determine the final score based on at least the feature score values.

The final score may then be used to confirm the character recognition once the character has first been recognised by e.g. comparing it to stored characters. A threshold value may be set so that if the final score is above this threshold, the character(s) can be confirmed to be correctly interpreted with a certain probability. The final score may also serve as an indicator how well the character(s) has/have been drawn by the user, i.e. the user's handwriting proficiency. The higher this score is, the better this user can handwrite or produce the requested characters or items more broadly or any sequence of them.

It is to be noted that the order of the steps shown in the flow chart of Figure 3 can be changed. Furthermore, e.g. steps 103 and 105 are optional if the feature importance values are not used in the determination of the final score. The method may also comprise a step where the data processing device 7 requests the user to enter a given set of characters. In this case, the data processing device 7 may visually show the second set of characters to be reproduced by the user and/or an acoustic announcement may be given. The user is advantageously from the same target group as the sample givers used to train the RF model. In this non-limiting example, the set of characters consists of the 26 letters of the English alphabet, i.e. letters a to z, and ten natural numbers, i.e. numbers 0 to 9. However, the teachings of the present invention are not limited to this particular set of characters. Different alphabets could be used instead, including Arabic, Greek, Latin, Cyrillic, Hebrew etc.

As mentioned above, the final score may optionally be compared with one or more threshold values. A further action may then be taken depending on whether or not the final score is below a given threshold value. The further action may for instance be a teaching or learning related action implemented by a teaching apparatus, which may comprise the data processing device 7. The final score thus allows categorising handwriting quality based on the detected quality at a feature level or at an overall level (i.e. the at the final score level). For instance, the final score allows determining a given number (e.g. from 1 to 10 or from 1 to 4 or any other suitable number of categories) of handwriting difficulty categories. The threshold for each category can be chosen as desired. Furthermore, one or more threshold values may vary over time as more data are collected and the algorithm retrained.

If the final application is used to train the user, then the application determines where the user is experiencing most difficulties and suggests specific adapted remediation activities tailored to specifically improve the features where the user is having difficulties. Based on the feature scores and/or the final score, a user handwriting profile can be extracted. This handwriting profile may be used to determine what kind of action is needed next, if any. For example, a first user may have specific problems in controlling the pressure they apply, while a second user may have problems with the shakiness, and a third user may have difficulties to keep a constant velocity. With this information about the user's handwriting, the application can provide an adapted remediation, specifically targeting their difficulties.

The above described method may be carried out by suitable circuits or circuitry. The terms "circuits" and "circuitry" refer to physical electronic components or modules (e.g. hardware), and any software and/or firmware ("code") that may configure the hardware, be executed by the hardware, and or otherwise be associated with the hardware. The circuits may thus be operable to carry out or they comprise means for carrying out the required method as described above.

While the invention has been illustrated and described in detail in the drawings and foregoing description, such illustration and description are to be considered illustrative or exemplary and not restrictive, the invention being not limited to the disclosed embodiment. Other embodiments and variants are understood, and can be achieved by those skilled in the art when carrying out the claimed invention, based on a study of the drawings, the disclosure and the appended claims. The invention may further relate to a computer program product comprising instructions for implementing at least some of the steps of the method when loaded and run on computing means of an electronic device.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used.

**Claims**

1.  A computer-implemented method of analysing one or more hand-drawn or handwritten items generated by a user, the method comprising:

    • collecting (107) one or more hand-drawn or handwritten items of the user, the user being part of a given user group;
    • extracting (109) a set of features from the one or more items, and allocating (109) a numerical value for a respective feature in the set of features, the respective feature in the set of features characterising a respective aspect of the one or more hand-drawn or handwritten items and/or its/their generation;
    • determining (111) one or more feature specific feature mean values and feature distribution related parameter values for the set of features of group members of the given user group;
    • determining (113) one or more feature score values for the set of features, a respective feature score value depending on a respective feature value, a respective feature mean value and a respective feature distribution related parameter value; and
    • determining (115) a final score for the user from the one or more feature score values.

2.  The method according to claim 1, wherein the determination of the feature score values comprises determining a feature specific deviation value, *deviation,* indicating how much a respective feature value deviates from a respective average feature value within the group members of the given user group as follows: $deviation = |(feature - f_{mean}(age))/f_{spread}(age)|$, where *feature* indicates the respective feature value, $f_{mean}(age)$ is a function describing a respective feature mean value as a function of age, and $f_{distribution}(age)$ is a function describing a respective feature distribution related parameter as a function of age, and obtaining the respective feature score, *score,* as $score = f(deviation)$, where *f* is a mathematical function.

3.  The method according to claim 2, wherein *f* is an exponential function such that $score = e^{(deviation)}$.

4.  The method according to claim 3, wherein the respective feature score is obtained as $score = e^{a \times deviation}$, where *a* denotes a negative real number.

5.  The method according to any one of the preceding claims, wherein a respective feature distribution related parameter value is a respective standard deviation value or a respective variance value for the respective features of the set of features of the group members of the given user group.

6.  The method according to any one of the preceding claims, wherein the final score is obtained as a weighted average or weighted sum of the feature score values, where the feature score values are weighted according to their importance value indicating their importance for a hand-drawn or handwritten item analysis.

7.  The method according to claim 6, wherein the importance values are obtained from an artificial intelligence system.

8.  The method according to claim 7, wherein obtaining the importance values comprises:

    • defining (101) hand-drawing and/or handwriting features;
    • training (103) the artificial intelligence system by using training data **characterised by** the hand-drawn and/or handwritten features; and
    • extracting (105) feature importance values from the artificial intelligence system.

9.  The method according to claim 7 or 8, wherein the artificial intelligence system comprises a random forest model.

10. The method according to any one of the preceding claims, wherein the features characterise at least one of the following hand-drawing and/or handwriting aspects: a static aspect relating to a geometry of the one or more hand-drawn and/or handwritten items; a kinematic aspect relating to dynamics of a hand-drawn and/or handwritten path generated by the one or more hand-drawn and/or handwritten items; a pressure related aspect relating to a pressure on a writing surface (5, 7) caused by a writing instrument (3) used to produce the one or more hand-drawn and/or handwritten items, a tilt related aspect relating to a tilt angle of the writing instrument (3) with respect to the writing surface (5, 7).

11. The method according to any one of the preceding claims, wherein the given user group is defined by at least one of the following group member related parameters: age, gender and handedness of the sample givers, and wherein the user is fully or partially defined by the parameters.

12. The method according to any one of the preceding claims, wherein the one or more items comprise letters of an alphabet and/or numerical digits.

13. The method according to any one of the preceding claims, wherein the method further comprises defining a final score threshold value, and carrying out one of the following operations:

> • confirming item recognition only if the final score has a first predefined relationship with respect to the final score threshold value;
> • proposing a handwriting exercise for the user if the final score has a second pre-defined relationship with respect to the final score threshold value; or
> • granting access to a restricted domain only if the final score has a third pre-defined relationship with respect to the final score threshold value.

14. The method according to claim 13, wherein the handwriting exercise is directed to a feature whose quality is determined not to satisfy a feature score threshold value.

15. A data processing device (7) for analysing one or more hand-drawn or handwritten items generated by a user, the device (7) comprising means for:

> • collecting one or more hand-drawn or handwritten items of the user, the user being part of a given user group;
> • extracting a set of features from the one or more items, and allocating a numerical value for a respective feature in the set of features, the respective feature in the set of features characterising a respective aspect of the one or more hand-drawn or handwritten items and/or its/their generation;
> • determining one or more feature specific feature mean values and feature distribution related parameter values for the set of features of group members of the given user group;
> • determining one or more feature score values for the set of features, a respective feature score value depending on a respective feature value, a respective feature mean value and a respective feature distribution related parameter value; and
> • determining a final score for the user from the one or more feature score values.

1

3

5

7

**Fig. 1**

13

9

15

11

7

**Fig. 2**

**Fig. 3**

Flowchart:

101 — Define handwriting features

103 — Train artificial intelligence system by using training data

105 — Extract feature importance values from artificial intelligence system

107 — Collect handwritten characters

109 — Extract features from handwritten characters and allocate numerical values for features

111 — Determine feature specific feature mean and standard deviation values based on pre-collected samples from users

113 — Determine feature score values based on numerical feature values, feature mean values and standard deviation values

115 — Determine final score based on feature score values

End

**Fig. 4**

Europäisches Patentamt
European Patent Office
Office européen des brevets

## EUROPEAN SEARCH REPORT

Application Number

EP 19 20 8146

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MEKYSKA JIRI ET AL: "Identification and Rating of Developmental Dysgraphia by Handwriting Analysis", IEEE TRANSACTIONS ON HUMAN-MACHINE SYSTEMS, vol. 47, no. 2, 1 April 2017 (2017-04-01), pages 235-248, XP011642744, IEEE, PISCATAWAY, NJ, USA ISSN: 2168-2291, DOI: 10.1109/THMS.2016.2586605 [retrieved on 2017-03-13] * Section II.A * * Section II. E.4) * * Section F. * * Section IV. * * Section V. * * tables III, IV * * abstract * * figure 1 * * Section II.B * | 1-15 | INV. G06K9/00 A61B5/16 G06K9/62 G06K9/03 |
| X | US 6 454 706 B1 (PULLMAN SETH L [US]) 24 September 2002 (2002-09-24) * column 4, line 49 - line 63 * * column 9, line 1 - line 28 * * column 19, line 40 - column 21 * * table 4 * | 1-5, 10-13,15 | TECHNICAL FIELDS SEARCHED (IPC) G06K A61B |
| X | US 4 495 644 A (PARKS JOHN R [GB] ET AL) 22 January 1985 (1985-01-22) * column 1, line 5 - line 12 * * column 2, line 7 - column 3, line 31 * * column 9, line 8 - line 25 * * column 9, line 47 - line 66 * * column 10, line 20 - line 65 * * column 11, line 3 - line 31 * * table 1 * * figures 7,9 * | 1-5,10, 12,13,15 | |

-/--

The present search report has been drawn up for all claims

| Place of search | Date of completion of the search | Examiner |
|---|---|---|
| The Hague | 17 April 2020 | Moreno, Marta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches
Patentamt

European
Patent Office

Office européen
des brevets

**EUROPEAN SEARCH REPORT**

Application Number

EP 19 20 8146

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2010/052708 A1 (UNIV CARMEL HAIFA ECONOMIC COR [IL]; ROSENBLUM SARA [IL]) 14 May 2010 (2010-05-14) * abstract * * page 3, line 3 - line 28 * * page 4, line 1 - line 10 * * page 4, line 25 - line 30 * * page 7, line 13 - line 30 * * page 31, line 23 - page 34, line 27 * ----- | 1,5, 10-15 | |
| A | THIBAULT ASSELBORN ET AL: "Automated human-level diagnosis of dysgraphia using a consumer tablet", NPJ DIGITAL MEDICINE, vol. 1, no. 1, 31 August 2018 (2018-08-31) , XP055686291, DOI: 10.1038/s41746-018-0049-x * the whole document * ----- | 1,6-15 | |
| A | DIAZ MOISES ET AL: "Dynamically enhanced static handwriting representation for Parkinson's disease detection", PATTERN RECOGNITION LETTERS,, vol. 128, 27 August 2019 (2019-08-27), pages 204-210, XP085915253, ELSEVIER, AMSTERDAM, NL ISSN: 0167-8655, DOI: 10.1016/J.PATREC.2019.08.018 [retrieved on 2019-08-27] * Section 1.2 * * Section 2. * * Section 4.1.2 * * Section 4.2.3 * * table 2 * ----- -/-- | 1,5-15 | TECHNICAL FIELDS SEARCHED (IPC) |

The present search report has been drawn up for all claims

| Place of search | Date of completion of the search | Examiner |
|---|---|---|
| The Hague | 17 April 2020 | Moreno, Marta |

EPO FORM 1503 03.82 (P04C01)

Europäisches
Patentamt

European
Patent Office

Office européen
des brevets

**EUROPEAN SEARCH REPORT**

Application Number

EP 19 20 8146

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BAO LY VAN ET AL: "On Using the Viterbi Path Along With HMM Likelihood Information for Online Signature Verification", IEEE TRANSACTIONS ON SYSTEMS, MAN AND CYBERNETICS. PART B:CYBERNETICS, vol. 37, no. 5, 1 October 2007 (2007-10-01), pages 1237-1247, XP011192468, IEEE SERVICE CENTER, PISCATAWAY, NJ, US ISSN: 1083-4419, DOI: 10.1109/TSMCB.2007.895323 * Section III.D * ----- | 1-4,15 | |

TECHNICAL FIELDS
SEARCHED      (IPC)

The present search report has been drawn up for all claims

| Place of search | Date of completion of the search | Examiner |
|---|---|---|
| The Hague | 17 April 2020 | Moreno, Marta |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 19 20 8146

This annex lists the patent family members relating to the patent documents cited in  the above-mentioned European search report.
The members are as contained in the European Patent Office EDP file on
The European Patent Office is in no way liable for these particulars which are merely given for the purpose of information.

17-04-2020

| Patent document cited in search report | | Publication date | Patent family member(s) | | Publication date |
|---|---|---|---|---|---|
| US 6454706 | B1 | 24-09-2002 | NONE | | |
| US 4495644 | A | 22-01-1985 | NONE | | |
| WO 2010052708 | A1 | 14-05-2010 | EP | 2352427 A1 | 10-08-2011 |
| | | | US | 2011217679 A1 | 08-09-2011 |
| | | | WO | 2010052708 A1 | 14-05-2010 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BJOERN H MENZE et al.** A comparison of random forest and its Gini importance with standard chemometric methods for the feature selection and classification of spectral data. *BMC Bioinformatics,* 2009, vol. 10